# EUROPEAN PATENT SPECIFICATION

(11) **EP 4 280 981 B1**
(45) Date of publication and mention of the grant of the patent: **12.03.2025**
(21) Application number: 21835431.4
(22) Date of filing: 16.12.2021
(51) Int. Cl.: A61B 17/34, A61M 16/04, A61M 16/08

(54) **SURGICAL INSTRUMENT**
CHIRURGISCHES INSTRUMENT
INSTRUMENT CHIRURGICAL

(30) Priority: 25.01.2021 NL 2027377
(43) Date of publication of application: 29.11.2023
(73) Proprietor: ProVinci Sabeair B.V., 2565 WN The Hague (NL)
(72) Inventor: HOREMAN-FRANSE, Tim, 2565 WN The Hague (NL); CEFAI, David, 2565 WN The Hague (NL)
(74) Representative: van Breda, Jacobus
(86) International application number: PCT/NL2021/050768
(87) International publication number: WO 2022/158967

(56) References cited:
- US-A- 5 374 252
- US-A1- 2013 310 750

## Description

The invention relates to a surgical instrument for piercing into a human or animal body, said instrument comprising an inner portion which is movably arranged within a needle-like outer portion, wherein said outer portion connects to the inner portion through a spring so as to provide with the outer portion during use a load on the inner portion, wherein said outer portion is provided with a proximal sleeve that provides a finger grip to a surgeon.

A typical example of such a surgical instrument is a Veress needle, hereinafter referred to as VN. The known VN is used to gain initial access to a peritoneal cavity of a patient to establish pneumoperitoneum. The use of the VN involves a blind insertion into the peritoneal cavity of the patient.

The use of the VN is probably popular because of its simplicity and effectiveness. It involves making a small incision in or near the umbilicus or in the left upper quadrant of the abdomen and then, in a blind fashion, putting the needle through the subcutaneous tissue, abdominal wall and the parietal peritoneum into the abdominal cavity. The VN technique is based on the ability of its blunt inner stylet to spring forward (since it is spring loaded) and to cover the sharp bevelled tip outer cannula when resistance diminishes after all tissue layers are passed. However, the surgeon cannot totally rely on this mechanism and therefore needs to develop a sense of the appropriate angle of insertion and the appropriate force to successfully puncture through the abdominal wall without overshooting into the underlying organs. The risk of damaging the underlying tissue with the tip of the cannula becomes high, when the reaction force that is generated by the abdominal wall drops to nearly zero (inside the abdominal cavity) in an instant. This immediate loss of resistance on the tip of the VN after puncturing causes acceleration of the needle towards the underlying tissues due to the slow reaction of the human control system, absence of stiff lower arm/hand support and relatively large mass of the surgeon's arm. Therefore, skilled and safe use of the VN requires a long learning curve to achieve the best possible instrument handling to prevent overshoot. Moreover, every patient's abdomen presents unique operating conditions, of which the specifics are unknown to the surgeon prior to the operation. These include the presence of adhesions, positions of the underlying tissues and viscera and the thickness of the abdominal wall.

In the prior art different proposals exist to counter the above mentioned problem of overshooting.

US2015/0265777 proposes an instrument wherein the surgeon sets a maximal insertion depth. Unfortunately, the tissue layer thickness is not always known and layers are flexible making adjustment of the insertion depth difficult.

US 5,364,365 allows overshooting but fixates the Veress mechanism as soon as it shoots in position. It seems however possible that even a blunt stylet with locked outer cannula can easily damage internal structures.

The articles by Nevler, A., Har-Zahav, G., Rosin, D., & Gutman, M. (2016); Safer trocar insertion for closed laparoscopic access: ex vivo assessment of an improved Veress needle. Surgical endoscopy, 30(2), 779-782, and by DuBois, K., Ryan, P., & Joanis, M. (2019). The Theia Soteria: Alternative Design for Safer Initial Entry During Laparoscopic Procedures focus on expanding the blunt area of the tip directly after insertion in order to reduce the stress when the tip hits underlying organs or structures.

The articles by Schaufler, A., Sühn, T., Esmaeili, N., Boese, A., Wex, C., Croner, R., ... & Illanes, A. (2019). Forcematic differentiation between Veress needle events in laparoscopic access using proximally attached audio signal characterization. Current Directions in Biomedical Engineering, 5(1), 369-371, and by Schaller, G., Kuenkel, M., & Manegold, B. C. (1995). The optical" Veress-needle"--initial puncture with a minioptic. Endoscopic surgery and allied technologies, 3(1), 55-57, report on the possibility to acquire information about intracorporeal tissue tool interactions of the VN tip, utilizing acoustic emissions or optic information recorded at the extracorporeal end of the needle.

The article by Greenberg, J. A. (2008). LapCap™. Reviews in Obstetrics and Gynecology, 1(2), 84 teaches to create a vacuum cup around the VN. When sucking the air out of the cup the abdominal wall is lifted away from the critical structures when the VN penetrates the tissue. A disadvantage here is that the surgeon loses manipulation flexibility as the configuration of the needle is fixed.

Finally, overshooting can be prevented by introducing a faster control system. This can be done by linking a robot arm with haptic sensation to the VN's body that generates the driving force, as is suggested by Nillahoot, N., & Suthakorn, J. (2013, December). Development of Veress needle insertion robotic system and its experimental study for force acquisition in soft tissue. In 2013 IEEE International Conference on Robotics and Biomimetics (ROBIO) (pp. 645-650). IEEE.

Although some of the proposals show interesting results, the complex nature of the proposed systems and the impact on workflow jeopardizes broader acceptance. It is therefore an object of the invention to provide a surgical instrument which is devoid of the complications of the known proposals, and which impairs the surgeon's operations as little as possible, so as to keep close to the surgeon's existing operational practices.

According to the invention a surgical instrument is proposed according to one or more of the appended claims. It is noted that although the previous discussion concentrates on the application of a VN, as the above recited preamble indicates the invention has broader application and relates to any surgical instrument for piercing into a human or animal body, wherein said instrument comprises an inner portion which is movably arranged within an needle-like outer portion, wherein said outer portion connects to the inner portion through a spring so as to provide with the outer portion during use a load on the inner portion, and wherein said outer portion is provided with a proximal sleeve that provides a finger grip to a surgeon. Examples thereof are for instance direct sharp trocar entries during laparoscopic surgery, and chest tube thoracostomies emergency or elective surgical airway.

US2013/0310750 discloses an instrument with features according to the preamble of claim 1, i.e. wherein the sleeve is releasably connectable to the outer portion and the instrument is provided with a release mechanism for releasing the sleeve from the outer portion, which release mechanism is actuable by motion of the inner portion.

In the instrument of the invention the outer portion is provided with a proximal ring or edge, and the sleeve is provided with a displaceable hook for hooking behind the ring or edge and thus provide a connection between the outer portion and the sleeve, wherein the release mechanism can be operable on the hook to remove it from its position behind the ring or edge. This makes possible that when the sleeve is used by the surgeon or a robot to push and insert the outer portion in the peritoneal cavity of a patient, the sudden drop of resistance when the insertion is accomplished and which is followed by an immediate forward movement of the spring-loaded inner portion, will have the effect that the inner portion activates the release mechanism which then neutralizes the driving force that the surgeon or robot applies to the sleeve, due to its release from the outer portion.

Suitably the release mechanism is mounted proximal on the inner portion and is arranged to operate on the hook of the sleeve when the inner portion moves towards the sleeve so as to release the sleeve from the outer portion.

The construction of the surgical instrument of the invention can be operationally effective and still be provided at limited cost when the release mechanism comprises a slanting first contact surface that is tailored to a correspondingly slanting second contact surface that is provided on the hook.

It is preferred that the hook is resiliently mounted on the sleeve so as to provide that the hook is displaced from its position behind the ring or edge of the outer portion at the time that the slanting first contact surface of the release mechanism impacts the second contact surface that is provided on the hook.

Suitably the release mechanism comprises a lever arm which is mounted proximal on the inner portion.

Optionally the proximal sleeve of the instrument is provided with an extension comprising one of a breathing tube or trocar tube. This relates to using the surgical instrument of the invention for intubating the trachea or intubating the abdominal wall with a trocar, respectively.

The invention will hereinafter be further elucidated with reference to the drawing of an exemplary embodiment of a surgical instrument according to the invention that is not limiting as to the appended claims.

In the drawing:
- figure 1 shows a Veress needle according to the prior art;
- figure 2 shows a Veress needle according to the invention;
- figure 3 shows a detail of the proximal portion of the Veress needle shown in figure 2; and
- figure 4 and 5 show the surgical instrument of the invention in an application for intubating the trachea or intubating the abdominal wall with a trocar, respectively.

Whenever in the figures the same reference numerals are applied, these numerals refer to the same or similar parts.

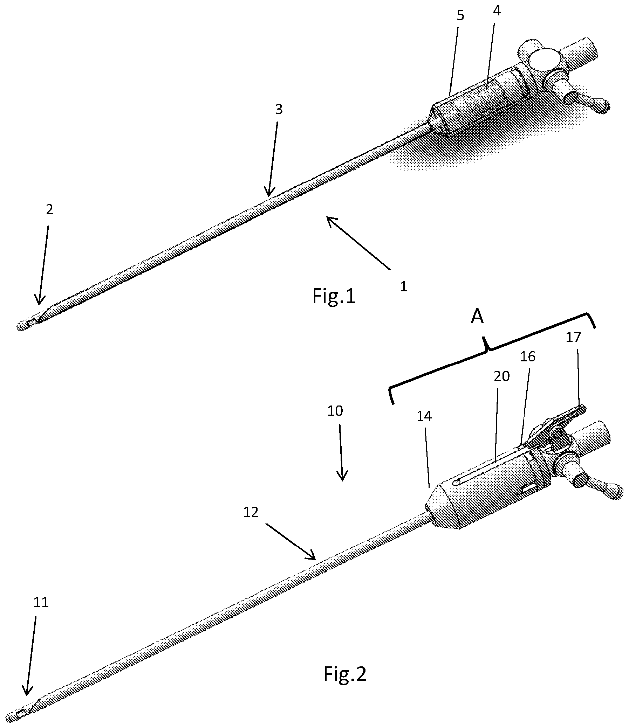

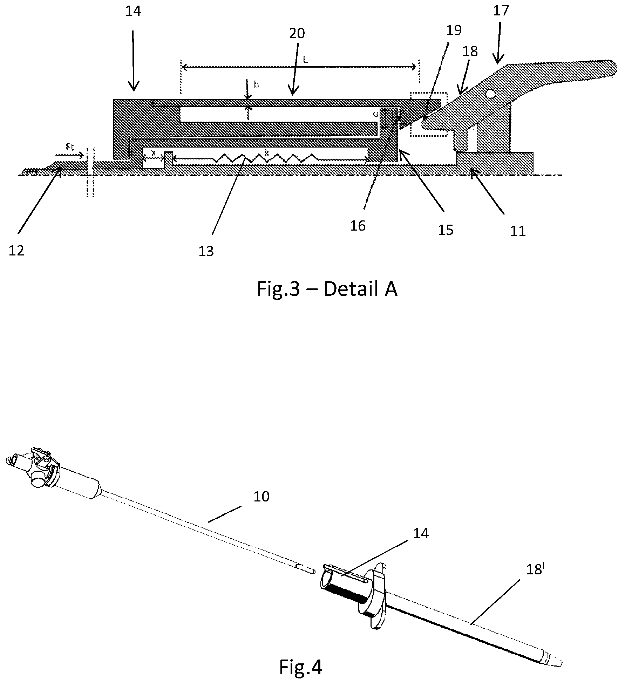

A Veress needle as shown in the figures is used for piercing into a human or animal body. As shown in figure 1 and in figure 2 the instrument 1, 10 comprises an inner portion 2, 11 which is movably arranged within a needle-like outer portion 3, 12, wherein said outer portion 3, 12 connects to the inner portion 2, 11 through a spring. In the prior art instrument 1 the spring is depicted with reference 4. The detail A of the proximal portion of the instrument 10 of the invention which is shown in figure 3, shows the spring as being depicted with reference 13.

Both in the prior art instrument 1 and in the instrument 10 of the invention, the spring serves to apply with the outer portion 3, 12 during use a load on the inner portion 2, 11. In connection therewith said outer portion 3, 12 is provided with a proximal sleeve 5, 14 that provides a finger grip for a surgeon. So far the prior art instrument 1 and the instrument 10 of the invention have corresponding features, albeit that the construction of the proximal sleeve 5 of instrument 1 differs from the proximal sleeve 14 of instrument 10.

The instrument 10 of the invention differentiates from the prior art in that the sleeve 14 is releasably connectable to the outer portion 12 and that the instrument 10 is provided with a release mechanism for releasing the sleeve 14 from the outer portion 12, which release mechanism is actuable by motion of the inner portion 11. The manner in which this can be preferably embodied is further explained with reference to the detail of the instrument of the invention as depicted in figure 3.

In figure 3 it is shown that the outer portion 12 is provided with a proximal ring or edge 15, and that the sleeve 14 is provided with a displaceable hook 16 for hooking behind the ring or edge 15 and thus provide a connection between the outer portion 12 and the sleeve 14. The release mechanism 17, which comprises a lever arm mounted proximal on the inner portion 11 can then be operable on the hook 16 to remove it from its position behind the ring or edge 15 according to the following elucidation.

The release mechanism 17 is mounted proximal on the inner portion 11 and is arranged to operate on the hook 16 of the sleeve 14 when the inner portion 11 moves towards the sleeve 14 so as to release the sleeve 14 from the outer portion 12. To that end it is preferable that the release mechanism 17 comprises a slanting first contact surface 18 that is tailored to a correspondingly slanting second contact surface 19 that is provided on the hook 16. The hook 16 is resiliently mounted on the sleeve 14 by means of the resilient support 20 so as to provide that the hook 16 can be displaced from its position behind the ring or edge 15 of the outer portion 12 at the time that the slanting first contact surface 18 of the release mechanism 17 impacts the second contact surface 19 that is provided on the hook 16.

Turning now to figure 4, the surgical instrument of the invention is shown wherein the proximal sleeve 14 is provided with an extension in the form of a breathing tube 18'. This construction is used for intubating the trachea. After piercing, all parts of the instrument 10 except for the sleeve 14 with the breathing tube 18' shoot backwards and can be removed from the surgical site. Than the proximal sleeve 14 is removed from the breathing tube 18 that remains in place for breathing.

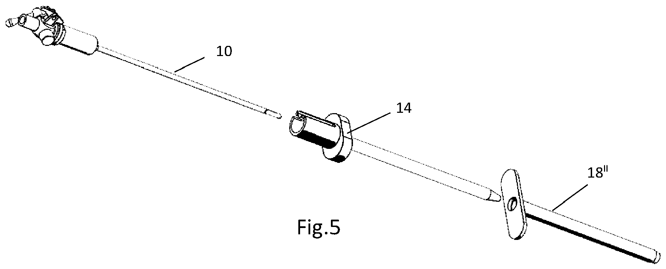

Figure 5 shows another application which is used for piercing the abdominal wall with a trocar. After piercing, all parts of the instrument 10 except for the sleeve 14 with an extension embodied as a trocar tube 18'' shoot backwards and can be removed from the surgical site. Then the proximal sleeve 14 is removed from the trocar tube 18'' that remains in place for instrument guidance towards the abdominal cavity.

Although the invention has been discussed in the foregoing with reference to an exemplary embodiment of the surgical instrument of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

## Claims

1. A surgical instrument (10) for piercing into a human or animal body, said instrument (10) comprising an inner portion (11) which is movably arranged within an needle-like outer portion (12), wherein said outer portion (12) connects to the inner portion (11) through a spring (13) so as to provide with the outer portion (12) during use a load on the inner portion (11), wherein said outer portion (12) is provided with a proximal sleeve (14) that provides a finger grip to a person, usually a surgeon, wherein the sleeve (14) is releasably connectable to the outer portion (12) and the instrument (10) is provided with a release mechanism (17) for releasing the sleeve (14) from the outer portion (12), which release mechanism (17) is actuable by motion of the inner portion (11), **characterized in that** the outer portion (12) is provided with a proximal ring or edge (15), and that the sleeve (14) is provided with a displaceable hook (16) for hooking behind the ring or edge (15) and thus provide a connection between the outer portion (12) and the sleeve (14), wherein the release mechanism (17) can be operable on the hook (16) to remove it from its position behind the ring or edge (15).

2. The surgical instrument of claim 1, **characterized in that** the release mechanism (17) is mounted proximal on the inner portion (11) and is arranged to operate on the hook (16) of the sleeve (14) when the inner portion (11) moves towards the sleeve (14) so as to release the sleeve (14) from the outer portion (12).

3. The surgical instrument of claim 1 or 2, **characterized in that** the release mechanism (17) comprises a slanting first contact surface (18) that is tailored to a correspondingly slanting second contact surface (19) that is provided on the hook (16).

4. The surgical instrument of claim 3, **characterized in that** the hook (16) is resiliently mounted on the sleeve (14) so as to provide that the hook (16) is displaced from its position behind the ring or edge (15) of the outer portion (12) at the time that the slanting first contact surface (18) of the release mechanism (17) impacts the second contact surface (19) that is provided on the hook (16).

5. The surgical instrument of any one of claims 1 - 4, **characterized in that** the release mechanism (17) comprises a lever arm which is mounted proximal on the inner portion (11).

6. The surgical instrument of any one of claims 1 - 5, **characterized in that** the proximal sleeve (14) is provided with an extension comprising one of a breathing tube (18') or trocar tube (18").

## Patentansprüche

1. Chirurgisches Instrument (10) zum Einstechen in einen menschlichen oder tierischen Körper, wobei das Instrument (10) einen inneren Abschnitt (11) umfasst, der beweglich in einem nadelartigen äußeren Abschnitt (12) angeordnet ist, wobei der äußere Abschnitt (12) mit dem inneren Abschnitt (11) durch eine Feder (13) verbunden ist, um mit dem äußeren Abschnitt (12) während der Verwendung eine Last auf den inneren Abschnitt (11) aufzubringen, wobei der äußere Abschnitt (12) mit einer proximalen Hülse (14) versehen ist, die einer Person, üblicherweise einem Chirurgen, einen Fingergriff bietet, wobei die Hülse (14) lösbar mit dem äußeren Abschnitt (12) verbunden werden kann und das Instrument (10) mit einem Freigabemechanismus (17) zum Loslösen der Hülse (14) von dem äußeren Abschnitt (12) versehen ist, wobei der Freigabemechanismus (17) durch Bewegung des inneren Abschnitts (11) betätigbar ist, **dadurch gekennzeichnet, dass** der äußere Abschnitt (12) mit einem proximalen Ring oder Rand (15) versehen ist und dass die Hülse (14) mit einem verschiebbaren Haken (16) versehen ist, um hinter dem Ring oder Rand (15) einzuhaken und somit eine Verbindung zwischen dem äußeren Abschnitt (12) und der Hülse (14) herzustellen, wobei der Freigabemechanismus (17) auf den Haken (16) einwirken kann, um ihn aus seiner Position hinter dem Ring oder Rand (15) zu entfernen.

2. Chirurgisches Instrument nach Anspruch 1, **dadurch gekennzeichnet, dass** der Freigabemechanismus (17) proximal an dem inneren Abschnitt (11) angebracht und so angeordnet ist, dass er auf den Haken (16) der Hülse (14) einwirkt, wenn sich der innere Abschnitt (11) in Richtung der Hülse (14) bewegt, um die Hülse (14) von dem äußeren Abschnitt (12) zu lösen.

3. Chirurgisches Instrument nach Anspruch 1 oder 2, **dadurch gekennzeichnet, dass** der Freigabemechanismus 17) eine schräge erste Kontaktfläche (18) umfasst, die auf eine entsprechend schräge zweite Kontaktfläche 19), die an dem Haken (16) vorgesehen ist, zugeschnitten ist.

4. Chirurgisches Instrument nach Anspruch 3, **dadurch gekennzeichnet, dass** der Haken (16) elastisch an der Hülse (14) angebracht ist, um so vorzusehen, dass der Haken (16) aus seiner Position hinter dem Ring oder Rand (15) des äußeren Abschnitts (12) zu dem Zeitpunkt verschoben wird, an dem die schräge erste Kontaktfläche (18) des Freigabemechanismus (17) auf die zweite Kontaktfläche (19) einwirkt, die an dem Haken (16) vorgesehen ist.

5. Chirurgisches Instrument nach einem der Ansprüche 1 bis 4, **dadurch gekennzeichnet, dass** der Freigabemechanismus (17) einen Hebelarm umfasst, der proximal an dem inneren Abschnitt (11) angebracht ist.

6. Chirurgisches Instrument nach einem der Ansprüche 1 bis 5, **dadurch gekennzeichnet, dass** die proximale Hülse (14) mit einer Verlängerung versehen ist, die einen Beatmungsschlauch (18') oder einen Trokartubus (18'') umfasst.

## Revendications

1. Instrument chirurgical (10) pour percer un corps humain ou animal, ledit instrument (10) comprenant une partie intérieure (11) qui est agencée de manière mobile dans une partie extérieure de type aiguille (12), dans lequel ladite partie extérieure (12) est reliée à la partie intérieure (11) par l'intermédiaire d'un ressort (13) de manière à pourvoir la partie extérieure (12) pendant l'utilisation d'une charge sur la partie intérieure (11), dans lequel ladite partie extérieure (12) est pourvue d'un manchon proximal (14) qui fournit une prise pour le doigt à une personne, habituellement un chirurgien, dans lequel le manchon (14) peut être relié de manière libérable à la partie extérieure (12) et l'instrument (10) est pourvu d'un mécanisme de libération (17) pour libérer le manchon (14) de la partie extérieure (12), lequel mécanisme de libération (17) peut être actionné par le mouvement de la partie intérieure (11), **caractérisé en ce que** la partie extérieure (12) est pourvue d'un anneau ou bord (15) proximal, et que le manchon (14) est pourvu d'un crochet (16) déplaçable à accrocher derrière l'anneau ou le bord (15) et ainsi fournissent une liaison entre la partie extérieure (12) et le manchon (14), dans lequel le mécanisme de libération (17) peut opérer sur le crochet (16) pour le retirer de sa position derrière l'anneau ou le bord (15).

2. Instrument chirurgical selon la revendication 1, **caractérisé en ce que** le mécanisme de libération (17) est monté de manière proximale sur la partie intérieure (11) et est agencé pour opérer sur le crochet (16) du manchon (14) lorsque la partie intérieure (11) se déplace en direction du manchon (14) de manière à libérer le manchon (14) de la partie extérieure (12).

3. Instrument chirurgical selon la revendication 1 ou 2, **caractérisé en ce que** le mécanisme de libération (17) comprend une première surface de contact inclinée (18) qui est adaptée à une deuxième surface de contact inclinée (19) correspondante qui est prévue sur le crochet (16)

4. Instrument chirurgical selon la revendication 3, **caractérisé en ce que** le crochet (16) est monté de manière élastique sur le manchon (14) de manière à permettre d'obtenir que le crochet (16) soit déplacé de sa position derrière l'anneau ou le bord (15) de la partie extérieure (12) au moment auquel la première surface de contact inclinée (18) du mécanisme de libération (17) heurte la deuxième surface de contact (19) qui est prévue sur le crochet (16).

5. Instrument chirurgical selon l'une quelconque des revendications 1 à 4, **caractérisé en ce que** le mécanisme de libération (17) comprend un bras de levier qui est monté de manière proximale sur la partie intérieure (11).

6. Instrument chirurgical selon l'une quelconque des revendications 1 à 5, **caractérisé en ce que** le manchon proximal (14) est pourvu d'une extension comprenant un d'un tube respiratoire (18') ou tube de trocart (18'').
